# EUROPEAN PATENT SPECIFICATION

(11) **EP 3 922 620 B1**
(45) Date of publication and mention of the grant of the patent: **02.07.2025**
(21) Application number: 19914315.7
(22) Date of filing: 13.08.2019
(51) Int. Cl.: C05F 11/00, C07C 51/09, C07C 27/02, C05D 9/00

(54) **PROCESS FOR OBTAINING A LIQUID FERTILIZER FOR LEAVES AND A LIQUID FERTILIZER COMPOSITION FOR LEAVES**
VERFAHREN ZUR HERSTELLUNG EINES FLÜSSIGEN DÜNGEMITTELS FÜR BLÄTTER UND FLÜSSIGE DÜNGEMITTELZUSAMMENSETZUNG FÜR BLÄTTER
PROCÉDÉ D'OBTENTION DE FERTILISANT LIQUIDE FOLIAIRE ET COMPOSITION FERTILISANTE LIQUIDE FOLIAIRE

(30) Priority: 05.02.2019 BR 102019002365
(43) Date of publication of application: 15.12.2021
(73) Proprietor: Hattori, Hélio Akinaga, 04536-010 São Paulo (BR)
(72) Inventor: Hattori, Hélio Akinaga, 04536-010 São Paulo (BR)
(74) Representative: Werner & ten Brink
(86) International application number: PCT/BR2019/050338
(87) International publication number: WO 2020/160630

(56) References cited:
- WO-A1-2011/142918
- AU-A1- 2013 224 650
- BR-A2- 102015 013 644
- BR-A2- 102015 013 644
- BR-A2- 102017 008 263
- BR-A2- 102017 008 263
- CN-A- 1 260 121
- CN-A- 104 529 659
- CN-A- 109 279 965
- DE-A1- 10 205 297
- US-A- 2 228 985
- US-A- 2 228 985
- US-A1- 2011 282 084
- MONTES D'OCA M G ET AL: "Base/acid-catalyzed FAEE production from hydroxylated vegetable oils", FUEL, IPC SIENCE AND TECHNOLOGY PRESS , GUILDFORD, GB, vol. 90, no. 2, 1 February 2011 (2011-02-01), pages 912 - 916, XP027511064, ISSN: 0016-2361, [retrieved on 20101120], DOI: 10.1016/J.FUEL.2010.10.030
- D'OCA M. G. M. ET AL.: "Based/acid-catalyzed FAEE production from hydroxylated vegetable oils", FUELS, vol. 90, 2011, pages 912 - 916, XP027511064, DOI: 10.1016/j.fuel.2010.10.030
- BORSOTTI, G. ET AL.: "Synthesis of phosphatidylcholines containg ricinoleic acid", TETRAHEDRON, vol. 57, 2001, pages 10219 - 102227, XP085040559
- BATISTA, R. B.: "Efeito da aplicação de acidos graxos na via das lipoxigenases de plantas de soja", QUIMICA NOVA, vol. 25, no. 6, 2002, pages 914 - 920, XP055731552

## Description

### Technical field

The present invention refers to the area of Chemistry applied to agribusiness, more particularly to a product obtained from organic compounds that has application in the agricultural area as compost or fertilizer.

### State of the Art

Castor bean cake is obtained from the seeds of the generic name castor bean oilseed *(Ricinus digitatus Nor., Ricinus gibsoni Cf, Ricinus leucocarpus Bert., Ricinus hidrdus Bess),* and represents a by-product of the castor bean production chain, the main product being castor oil also called mammon oil. The term "mammon" originates from the term "quimbundo mumono", and thanks to the similarity of its leaves to the papaya tree, it was also influenced by the term "papaya".

Castor bean cake has been used around the world as an organic fertilizer to recover depleted land.

Castor oil is rich in fatty carboxylic acids, particularly in ricinoleic acid (80% to 90%), which provides good viscosity and low temperature solubility in alcohol.

Ricinoleic acid or 12-hydroxy-9-cis-octadecenoic acid is industrially extracted through saponification or fractional distillation of hydrolyzed castor oil.

The saponification reaction receives this name, due to being widely used in soap making. The reaction is characterized by the basic hydrolysis of lipids (or triglycerides, or vegetable oils, or fats), by adding a strong base and heating. Triglyceride molecules break down to form a glycerin molecule and three fatty carboxylic acid molecules in the form of a salt. If the base used is sodium, such as sodium hydroxide, the salt will be a sodium salt.

Fatty carboxylic acid salts have the property of dissolving in either polar media such as water or non-polar media. This is why the "soap" obtained at the end has the property of a surfactant.

The basic saponification reaction equation is shown below:

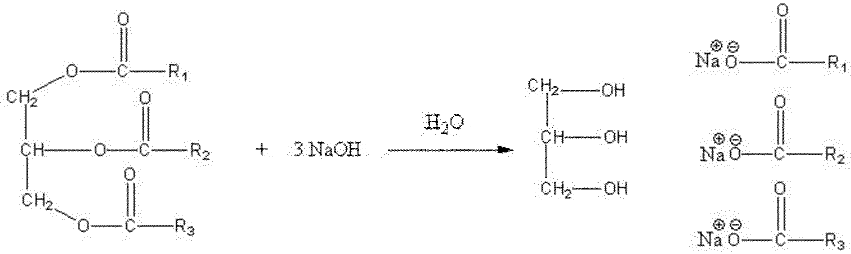

The use of ricinoleic acid in fertilizers is already known.

The document CN 105948980 refers to special fertilizer for grapes containing castor bean in its cake composition.

GB 1178849 relates to a granular fertilizer whose granulating agent can be a monoricinoleate or ricinoleic acid.

CN 104529659 describes a fertilizer that uses ricinoleic acid.

CN 106431644 teaches a selenium-rich fertilizer containing ricinoleic acid.

Patent application BR102017008963 discloses a liquid foliar fertilizer using fatty acids as raw materials, having as active principle the soluble esters of fatty acids. Conversely, the instant application fertilizer is based on ricinoleic acid saponified with sulfuric acid plus triethanolamine.

Patent document BR102015013664 discloses a composition comprising mammon (castor) oil and/or ricinoleic acid reacted with ethanolamine for use as a bactericidal and fungicidal product, having its main application in oilfield water disinfection; This document does not mention the use of the abovementioned composition as a foliar fertilizer; moreover it does not disclose the saponification of ricinoleic acid.

A paper by Montes D'Oca et al. titled "Base/acid catalyzed FAEE production from hydroxylated vegetable oils" (Fuel, vol 90, no.2, pages 912-916) refers to the production of Fatty Acid Ethyl Ester (FAEE) of castor oil, mainly comprising 80% - 90% ricinoleic acid, to yield a fuel, namely biodiesel. In the process described in this paper, the production of soap is an unwanted side reaction, while the object of the instant invention is the ricinoleic acid saponification, i.e., the production of soap. Besides, the end product disclosed in this paper does not comprise ethanolamine.

Patent document US2228985 relates to new products adapted for use as wetting, dispersing or emulsifying agents and a production method which comprises heating castor oil and triethanolamine to a temperature above 100 degrees Centigrade in order to produce ricinoleic acid ester of triethanolamine, and adding acetic acid to neutralize the reaction product. This document does not concern itself with the production of a fertilizer.

But none of the prior art documents teach a composition for liquid foliar fertilizer that is easy to apply and has good absorption properties by the plant.

### Invention Objects

The main object of the invention is to provide a product derived from ricinoleic acid with the addition of triethanolamine (C₆H₁₃NO₃) capable of being used as a liquid foliar fertilizer to accelerate vegetative growth.

Ricinoleic acid has inherent antibacterial and antifungal properties.

### General Description of the Invention

The present invention refers to a process for obtaining a liquid fertilizer for foliar application with a final formulation comprising:
- ricinoleic acid saponified with sulfuric acid;
- triethanolamine (C₆H₁₅NO₃);
- water.

The invention provides a means of accelerating vegetative growth through the proposed composition that has a group of fatty acids and amino acids essential to plant development.

The invention also provides foliar metabolic regulatory means without the presence of toxic components, being useful for foliar application in all kinds of plants.

### Description of the Figures

The invention will be better understood through the description of a preferred, nonlimiting exemplary embodiment, and the figures that refer to it, in which:
Figure 1 illustrates a way of foliar application of the fertilizer of the invention, by spraying the product on plants.
Figure 2 illustrates another way of applying the fertilizer product of the invention, by spraying on plants.

### Detailed Description of the Invention

The present invention refers to a process for obtaining a liquid fertilizer composition, for foliar application, comprising the following steps:
(a)in a first container dilute a strong base in water until reaching a mass concentration of about 5% to about 25% at a temperature between about 30 °C and about 50 °C and stir until complete dissolution;
(b)add castor oil in the ratio of about 70% to about 90% of the mass of item (a) above and an alcohol in the ratio of about 3% to about 12% of the mass of item (a) above, stirring strongly, leaving to rest and then stirring again;
(c) in a second container dilute sulfuric acid in water up to about 40% to about 65% by weight to a temperature of 0 °C;
(d)without stirring add to the mixture of the second container the mixture of the first container, followed by stirring;
(e) stop the stirring and leave to rest; when phase separation occurs drain the lower phase which will be discarded;
(f) to the product obtained in step (e) add water in an amount of about 30% to about 55% of the mass of item (a) above, stir and leave to rest; phase separation will occur, drain the lower phase (waste) which will be discarded;
(g) to the product obtained in the previous step add water in an amount of about 30% to about 55% of the mass of item (a) above, stir, and leave to rest; after phase separation, discard the lower phase;
(h) add triethanolamine in a container, then water and later the product obtained in the previous step (g), in the following ratio by mass:
   - 2 parts of ricinoleic acid saponified with sulfuric acid;
   - 1 part of triethanolamine (C₆H₁₅NO₃);
   - 2 to 10 parts of water.

The strong base used in step (a) can be an alkali metal hydroxide such as sodium hydroxide.

Alcohol can be an alcohol containing C2 to C6, such as ethanol.

Preferably, the process of obtaining a liquid fertilizer composition for foliar application of the invention comprises the following steps:
(a) in a container dilute 100% sodium hydroxide in flakes in water to reach a concentration of 13.04% by mass at a temperature between about 35°C and about 45 °C and stir until dissolved;
(b) add extra pale castor oil in the ratio of 82.61% of the mass of item (a) above and ethanol in the ratio of 6.52% of the mass of item (a) above, under stronger stirring for 15 min, leave to rest for 10 min and then stir again for 25 min;
(c) in another container dilute 98% sulfuric acid in water to reach a mass concentration of 52.27% at a temperature of about 0 °C;
(d) add to the mixture obtained in the previous step (c) to the mixture obtained in step (b), without stirring, and then stir for 5 min;
(e) turn off the stirring and leave to rest for 10 to 20 min, when phase separation will occur; then drain and discard the lower phase;
(f) to the product obtained in step (e) add water in an amount of 43.48% of the mass of item (a) above, stir for 15 min and leave to rest for 10 to 20 min (or the time required for phase separation); then drain and discard the lower phase;
(g) to the product obtained in the previous step, water is added in an amount of 43.48% of the mass of item (a) above; stir for 15 min, then leave to rest for 30 to 90 min, and then drain and discard the lower phase;
(h) pour triethanolamine in a container, then water and later the product obtained in the previous step (g), in the following ratios by mass:
   - 2 parts of ricinoleic acid saponified with sulfuric acid;
   - 1 part of triethanolamine (C₆H₁₃NO₃),
   - 2 to 10 parts of water.

Said ricinoleic acid is saponified with 98% sulfuric acid, thus receiving the name of Ricinoleic Sulfuric Acid. This process differs from the common process that uses hydrochloric acid.

The method according to the invention ultimately results in a fertilizer product for foliar application, the composition comprising the following compounds:
- ricinoleic acid saponified with sulfuric acid;
- triethanolamine (C₆H₁₅NO₃);
- water.

According to the invention, the composition comprises by mass, relative to the total mass of the composition:
- 2 parts of ricinoleic acid saponified with sulfuric acid;
- 1 part of triethanolamine 85% (C₆H₁₃NO₃),
- 2 to 10 parts of water.

This mixture of chemical compounds comprises a group of fatty acids and amino acids that are essential for plant development.

The composition according to the invention can also comprise other types of nutrients, known in the art, depending on the type of crop to be treated.

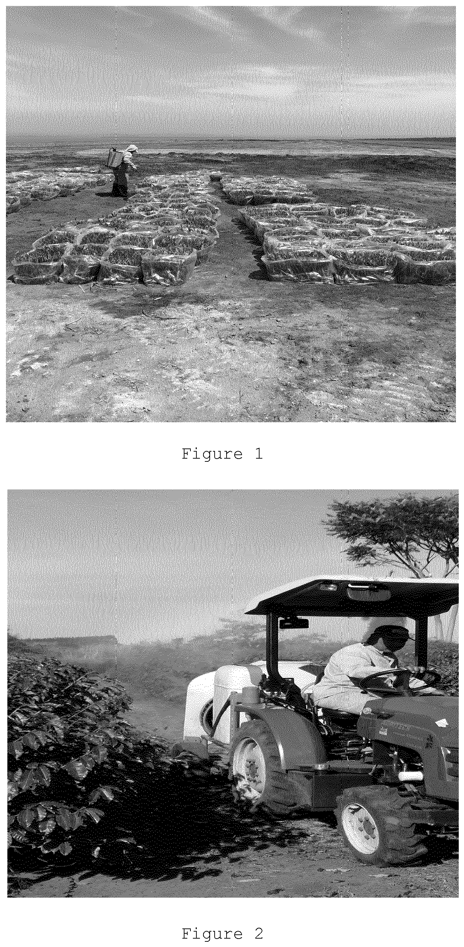

The final product, according to the invention, will be a liquid that can be applied by spraying onto the leaves of the plants, as shown in figures 1 and 2.

The dilution of the final product for application in sprayed crops can vary from 1/600 to 1/1200 depending on the crop.

Tests were carried out to prove the validity of the information mentioned above, and the improvement in productivity was confirmed.

The tests were carried out with different kinds of cultures, in order to verify the efficiency of the composition of the invention in the different crops.

The following examples are merely illustrative of the scope of the invention, and should not be used for limiting purposes.

### Example 1

Production process for 200 liters of ricinoleic acid saponified with sulfuric acid.

Raw materials used:

**[TABLE 1]**

| Substance | Amount |
|---|---|
| 100% Commercial Sodium Hydroxide in flakes | 30 kg |
| Extra pale castor oil | 190 kg |
| 98% sulfuric acid | 40 kg |
| Ethanol | 15 liters |
| Ice | 35 kg |
| Tap water | |

The process comprised the following steps:
1) Dilute the Sodium Hydroxide directly in the tank in 200 liters of warm water (35° to 45°C). Mix until dissolved.
2) Add Extra Pale Castor Oil.
3) Add ethanol.
4) Stir at high speed for 15 minutes.
5) Turn off the stirring and leave to rest for 10 minutes.
6) Stir at high speed for 25 minutes.
7) In a separate container, dilute the sulfuric acid in 35kg of ice.
8) With the stirrer off, add the sulfuric acid + ice mixture (item 7).
9) Stir for 5 minutes.
10) Stop stirring and wait for phase separation (10 to 20 minutes).
   *If phase separation does not occur within the indicated times, add sulfuric acid in batches of 5 kg, stir for 15 minutes and wait for separation.
11) Drain the lower phase (discard)
12) Add 100 liters of water.
13) Stir for 15 minutes.
14) Stop stirring and wait for phase separation (10 to 20 minutes).
   *If phase separation does not occur within the indicated times, add sulfuric acid in batches of 5 kg, stir for 15 minutes and wait for separation.
15) Drain the lower phase (discard).
16) Add 100 liters of water.
17) Stir for 15 minutes.
18) Stop stirring.
19) Wait for complete phase separation (30 to 90 minutes).
20) Completely drain the lower phase (discard).
21) At the end of this process about 200 l of ricinoleic acid saponified with sulfuric acid are obtained.

### Example 2

The ricinoleic acid saponified with sulfuric acid obtained in example 1, is used to produce a fertilizer in liquid form by adding triethanolamine and water in the ratios indicated below:
- 2 parts of ricinoleic acid saponified with sulfuric acid;
- 1 part of triethanolamine 85% (C₆H₁₃NO₃),
- 2 parts of water.

In the following examples, the fertilizer composition according to the invention, obtained by dilution in water at the ratio of 1/600 of product and water, respectively, was used.

### Example 3

The test was carried out with coffee crop, in the region of São Sebastião do Paraíso, State of Minas Gerais, on a private property, with an increase in productivity of approximately 14% in the amount of bags of coffee produced, related to control trees.

The amounts produced, both in the control trees and in the plants where the product was applied, can be seen in Table 2. The variety of coffee in which the tests were carried out is Mundo Novo. The crop in question is the crop harvested in July 2018.

**[Table 2]**

| Type of plant | Number of plants | Production (bags) | Production per 1000 plants (bags) |
|---|---|---|---|
| Plant with compound application | 6000 | 31.33 | 5.22 |
| control plant | 4000 | 18.34 | 4.59 |

It is important to emphasize that each bag of coffee weights 60 kilos and that the variety and age of the coffee crops are the same, both for the control planta and the treated plants.

### Example 4

The test was carried out in the Bauru region, in the State of São Paulo, also on a private property. The crop was orange.

The harvest is from November 2018. The values obtained in the tests can be seen in Table 3. These tests showed an increase in productivity of approximately 16% in the number of orange boxes produced by the treated trees compared to the control tree.

**[Table 3]**

| Type of plant | Number of plant (trees) | Production (boxes) | Production per 1 plant (boxes) |
|---|---|---|---|
| Plant with compound application | 988 | 1853 | 1.876 |
| control plant | 3504 | 5675.9 | 1.620 |

The tests performed were carried out with oranges of the same variety and age, both for the treated and the control trees. Each orange box used in the tests had a weight of 48 kilograms.

### Example 5

The test was carried out in the region of Barra Bonita, State of São Paulo, on another private property, in sugarcane crop, with pre-sprouted seedlings, of CTC 9001 variety.

The tests were carried out in November 2017. It was verified in this test an increase of 13% of dry vegetative mass in the treated seedlings, as compared with the control seedlings. The values obtained in the test can be seen in Table 4.

**[Table 4]**

| type of seedling | Number of seedlings (trays) | Quantity of seedlings (per tray) | Production (grams) |
|---|---|---|---|
| Seedlings with | 25 | 162 | 260.87 |
| compound application | | | |
| control seedlings | 25 | 162 | 230.90 |

The production values were collected by sampling, two trays of each type of seedling crop were collected (treated and control). The test was performed with crops of the same age and variety. The production values were measured after the drying period in a natural drying oven.

## Claims

1. **Process for obtaining a liquid foliar fertilizer** for spraying onto the leaves of plants wherein said process comprises the following steps:
(a) in a first container dilute a strong base in water until reaching a mass concentration of about 5% to about 25% at a temperature between about 30 °C and about 50 °C and stir until complete dissolution;
(b) add castor oil in the ratio of about 70% to about 90% of the mass of item (a) above and an alcohol in the ratio of about 3% to about 12% of the mass of item (a) above, stirring strongly, leaving to rest and then stirring again
(c) in a second container dilute sulfuric acid in water up to about 40% to about 65% by weight at a temperature of 0 °C;
(d) without stirring, add to the mixture of the second container the mixture of the first container, followed by stirring;
(e) stop the stirring and leave to rest; when phase separation occurs drain the lower phase and discard it;
(f) to the product obtained in step (e) add water in an amount of about 30% to about 55% of the mass of item (a) above, stir, and leave to rest; after phase separation drain the lower phase (waste) and discard it;
(g) to the product obtained in the previous step add water in an amount of about 30% to about 55% of the mass of item (a) above, stir, and leave to rest; after phase separation, discard the lower phase;
(h) add triethanolamine in a container, then water and later the product obtained in the previous step (g), in the following ratio by mass:
- 2 parts ricinoleic acid saponified with sulfuric acid;
- 1 part triethanolamine (C₆H₁₃NO₃),
- 2 to 10 parts water.

2. **Process** according to claim 1, wherein the strong base is sodium hydroxide.

3. **Process** according to claim 1, wherein the alcohol is ethanol.

4. **Process** according to claim 1, wherein said process comprises the following steps:
(a) in a container dilute 100% sodium hydroxide in flakes in water to reach concentration of 13.04% by mass at a temperature between about 35 °C and about 45 °C and stir until dissolved;
(b) add extra pale castor oil in the ratio of 82.61% of the mass of item (a) above and ethanol in the ratio of 6.52% of the mass of item (a) above, under stronger stirring for 15 min, leave to rest for 10 min and then stir again for 25 min;
(c) in another container dilute 98% sulfuric acid in water to reach a mass concentration of 52.27% at a temperature of about 0 °C;
(d) add to the mixture obtained in the previous step (c) to the mixture obtained in step (b), without stirring, and then stir for 5 min;
(e) turn off the stirring and leave to rest for 10 to 20 min; after phase separation drain and discard the lower phase;
(f) to the product obtained in step (e) add water in an amount of 43.48% of the mass of item (a) above, stir for 15 min and leave to rest for 10 to 20 min; after phase separation), drain and discard the lower phase;
(g) to the product obtained in the previous step, water is added in an amount of 43.48% of the mass of item (a) above; and stir for 15 min, then leave to rest for 30 to 90 min: after phase separation, drain and reject the lower phase;
(h) pour triethanolamine in a container, then water and later the product obtained in the previous step (g), in the following ratio by mass:
- 2 parts ricinoleic acid saponified with sulfuric acid;
- 1 part triethanolamine (C₆H₁₅NO₃) 85%;
- 2 to 10 parts water.

5. **Liquid fertilizer composition** for foliar application obtained by the process of claim 1, wherein said composition comprises:
- ricinoleic acid saponified with sulfuric acid;
- triethanolamine (C₆H₁₅NO₃);
- Water.

6. **Liquid fertilizer composition** according to claim 5, wherein said composition comprises:
- 2 parts ricinoleic acid saponified with sulfuric acid;
- 1 part triethanolamine 85% (C₆H₁₅NO₃);
- 2 to 10 parts water.

7. **Liquid fertilizer composition** according to claim 5, wherein said composition further comprises other types of nutrients suitable for each type of crop.

## Patentansprüche

1. **Verfahren zur Herstellung eines flüssigen Blattdüngers** zum Aufsprühen auf die Blätter von Pflanzen, wobei das Verfahren die folgenden Schritte umfasst:
(a) Verdünnen in einem ersten Behälter einer starken Base in Wasser, bis eine Massenkonzentration von etwa 5% bis etwa 20% bei einer Temperatur zwischen etwa 30°C und etwa 50°C erreicht ist, und Rühren bis zur vollständigen Auflösung;
(b) Hinzufügen von Rizinusöl im Verhältnis von etwa 70% bis etwa 90% der Masse von Punkt (a) und von einem Alkohol im Verhältnis von etwa 3% bis etwa 12% der Masse von Punkt (a), kräftig rühren, ruhen lassen und dann erneut rühren;
(c) Verdünnen in einem zweiten Behälter von Schwefelsäure in Wasser auf etwa 40 Gew.-% bis etwa 65 Gew.-% bei einer Temperatur von 0°C;
(d) Hinzufügen der Mischung aus dem ersten Behälter zur Mischung aus dem zweiten Behälter ohne zu Rühren und anschließendes Rühren;
(e) Beenden des Rührens und ruhenlassen; bei Eintreten der Phasentrennung die untere Phase abgießen und verwerfen;
(f) Hinzufügen von Wasser in einer Menge von etwa 30% bis etwa 55% der Masse von Punkt (a) zum in Schritt (e) erhaltenen Produkt, rühren und ruhen lassen; nach der Phasentrennung die untere Phase (Abfall) abgießen und verwerfen;
(g) Hinzufügen von Wasser in einer Menge von etwa 30% bis etwa 55% der Masse von Punkt (a) zum im vorhergehenden Schritt erhaltenen Produkt, rühren und ruhen lassen; nach der Phasentrennung die untere Phase verwerfen;
(h) einem Behälter Triethanolamin zugeben, dann Wasser und anschließend das im vorherigen Schritt (g) erhaltene Produkt im folgenden Massenverhältnis:
- 2 Teile mit Schwefelsäure verseifte Rizinolsäure;
- 1 Teil Triethanolamin (C₆H₁₅NO₃),
- 2 bis 10 Teile Wasser.

2. **Verfahren** gemäß Anspruch 1, wobei die starke Base Natriumhydroxid ist.

3. **Verfahren** gemäß Anspruch 1, wobei der Alkohol Ethanol ist.

4. **Verfahren** gemäß Anspruch 1, wobei das Verfahren die folgenden Schritte umfasst:
(a) Verdünnen von 100%igen Natriumhydroxid in Flocken in Wasser, um bei einer Temperatur zwischen etwa 35°C und etwa 45°C eine Konzentration von 13,04 Massenprozent zu erreichen und bis zur Auflösung rühren;
(b) Hinzufügen von extra hellem Rizinusöl im Verhältnis von 82,61% der Masse von Punkt (a) bei starkem Rühren für 15 min, Ruhenlassen für 10 und erneutem Rühren für 25 min;
(c) Verdünnen in einem anderen Behälter von 98% Schwefelsäure in Wasser, um eine Massenkonzentration von 52,27% bei einer Temperatur von etwa 0°C zu erhalten;
(d) Hinzufügen der im vorherigen Schritt (c) erhaltenen Mischung zur in Schritt (b) erhaltenen Mischung ohne zu rühren;
(e) Stoppen des Rührens und für 10 bis 20 min ruhen lassen; nach der Phasentrennung die untere Phase abgießen und verwerfen;
(f) Hinzufügen von Wasser in einer Menge von 43,48% der Masse von Punkt (a) zu dem im vorherigen Schritte erhaltenen Produkt, 15 min rühren und 10 bis 20 min ruhen lassen; nach der Phasentrennung die untere Phase abgießen und verwerfen;
(g) Hinzufügen von Wasser in einer Menge von 43,48% der Masse von Punkt (a) zu dem im vorherigen Schritte erhaltenen Produkt; und 15 min rühren, dann 30 bis 90 min ruhen lassen; nach der Phasentrennung die untere Phase abgießen und verwerfen;
(h) peinem Behälter Triethanolamin zugeben, dann Wasser und anschließend das im vorherigen Schritt (g) erhaltene Produkt im folgenden Massenverhältnis:
- 2 Teile mit Schwefelsäure verseifte Rizinolsäure;
- 1 Teil Triethanolamin (C₆H₁₅NO₃),
- 2 bis 10 Teile Wasser.

5. **Flüssige Düngemittelzusammensetzung** zur Blattanwendung, erhalten durch das Verfahren nach Anspruch 1, wobei die Zusammensetzung umfasst:
- mit Schwefelsäure verseifte Rizinolsäure;
- Triethanolamin (C₆H₁₅NO₃),
- Wasser

6. **Flüssige Düngemittelzusammensetzung** nach Anspruch 5, wobei die Zusammensetzung umfasst:
- 2 Teile mit Schwefelsäure verseifte Rizinolsäure;
- 1 Teil Triethanolamin 85%(C₆H₁₅NO₃),
- 2 bis 10 Teile Wasser.

7. **Flüssige Düngemittelzusammensetzung** nach Anspruch 5, wobei die Zusammensetzung außerdem andere Nährstoffarten umfasst, die für jede Art von Nutzpflanze geeignet sind.

## Revendications

1. **Procédé d'obtention d'un engrais foliaire liquide** destiné à être pulvérisé sur les feuilles de plantes, ledit procédé comprenant les étapes suivantes:
(a) dans un premier récipient, diluer une base forte dans de l'eau jusqu'à atteindre une concentration massique d'environ 5% à environ 25% à une température comprise entre environ 30°C et environ 50°C et remuer jusqu'à dissolution complète;
(b) ajouter de l'huile de ricin dans un rapport d'environ 70% à environ 90% de la masse de l'élément (a) ci-dessus et un alcool dans un rapport d'environ 3% à environ 12% de la masse de l'élément (a) ci-dessus, en remuant fortement, en laissant reposer puis en remuant à nouveau;
(c) dans un deuxième récipient, diluer l'acide sulfurique dans de l'eau jusqu'à environ 40% à environ 65% en poids à une température de 0°C;
(d) sans remuer, ajouter au mélange du deuxième récipient le mélange du premier récipient, puis remuer;
(e) arrêter de remuer et laisser reposer; lorsque la séparation de phase se produit, égoutter la phase inférieure et la jeter;
(f) au produit obtenu à l'étape (e), ajouter une quantité d'eau d'environ 30% à environ 55% de la masse de l'élément (a) ci-dessus, remuer et laisser reposer; après la séparation de phase, égoutter la phase inférieure (déchet) et la jeter;
(g) au produit obtenu à l'étape précédente, ajouter une quantité d'eau d'environ 30% à environ 55% de la masse de l'élément (a) ci-dessus, remuer et laisser reposer; après la séparation des phases, jeter la phase inférieure;
(h) ajouter de la triéthanolamine dans un récipient, puis de l'eau et plus tard le produit obtenu à l'étape précédente (g), dans le rapport massique suivant:
- 2 parts d'acide ricinoléique saponifié avec de l'acide sulfurique;
- 1 part de triéthanolamine (C₆H₁₅NO₃),
- 2 à 10 parts d'eau.

2. **Procédé** selon la revendication 1, dans lequel la base forte est de l'hydroxyde de sodium.

3. **Procédé** selon la revendication 1, dans lequel l'alcool est de l'éthanol.

4. **Procédé** selon la revendication 1, dans lequel ledit procédé comprend les étapes suivantes:
(a) dans un récipient, diluer 100% d'hydroxyde de sodium en flocons dans de l'eau pour atteindre une concentration de 13,04% en masse à une température comprise entre environ 35°C et environ 45°C et remuer jusqu'à dissolution;
(b) ajouter de l'huile de ricin extra pâle dans un rapport de 82,61% de la masse de l'élément (a) ci-dessus et de l'éthanol dans un rapport de 6,52% de la masse de l'élément (a) ci-dessus, en remuant plus fortement pendant 15 min, laisser reposer pendant 10 min puis remuer à nouveau pendant 25 min;
(c) dans un autre récipient, diluer de l'acide sulfurique à 98% dans de l'eau pour atteindre une concentration massique de 52,27 % à une température d'environ 0°C;
(d) ajouter le mélange obtenu à l'étape précédente (c) au mélange obtenu à l'étape (b), sans remuer, puis remuer pendant 5 min;
(e) arrêter de remuer et laisser reposer pendant 10 à 20 min; après la séparation des phases, égoutter et jeter la phase inférieure;
(f) au produit obtenu à l'étape (e) ajouter une quantité d'eau de 43,48 % de la masse de l'élément (a) ci-dessus, remuer pendant 15 min et laisser reposer pendant 10 à 20 min; après la séparation des phases, égoutter et jeter la phase inférieure;
(g) au produit obtenu à l'étape précédente, de l'eau est ajoutée à raison de 43,48 % de la masse de l'élément (a) ci-dessus; et on agite pendant 15 min, puis on laisse reposer pendant 30 à 90 min: après la séparation de phases, on égoutte et on rejette la phase inférieure;
(h) verser de la triéthanolamine dans un récipient, puis de l'eau et plus tard le produit obtenu à l'étape précédente (g), dans le rapport massique suivant:
- 2 parts d'acide ricinoléique saponifié avec de l'acide sulfurique;
- 1 part de triéthanolamine (C₆H₁₅NO₃) 85%;
- 2 à 10 parts d'eau.

5. **Composition d'engrais liquide** pour application foliaire, ladite composition comprenant:
- de l'acide ricinoléique saponifié à l'acide sulfurique:
- de la triéthanolamine (C₆H₁₅NO₃),
- de l'eau.

6. **Composition d'engrais liquide** selon la revendication 5, ladite composition comprenant:
- 2 parts d'acide ricinoléique saponifié avec de l'acide sulfurique;
- 1 part d triéthanolamine 85% (C₆H₁₅NO₃),
- 2 à 10 parts d'eau.

7. **Composition d'engrais liquide** selon la revendication 5, ladite composition comprenant en outre d'autres types de nutriments adaptés à chaque type de culture.
